# EUROPÄISCHE PATENTSCHRIFT

(11) **EP 0 977 509 B1**
(45) Veröffentlichungstag und Bekanntmachung des Hinweises auf die Patenterteilung: **12.06.2002**
(21) Anmeldenummer: 99907410.7
(22) Anmeldetag: 23.01.1999
(51) Int. Cl.: A61B 1/04

(54) **ENDOSKOP, INSBESONDERE VIDEO-ENDOSKOP**
ENDOSCOPE AND MORE PRECISELY VIDEO ENDOSCOPE
ENDOSCOPE, NOTAMMENT ENDOSCOPE VIDEO

(30) Priorität: 04.02.1998 DE 19804234
(43) Veröffentlichungstag der Anmeldung: 09.02.2000
(73) Patentinhaber: Karl Storz GmbH & Co. KG, 78532 Tuttlingen (DE)
(72) Erfinder: RUDISCHHAUSER, Jürgen, D-78532 Tuttlingen (DE); KEHR, Ulrich, D-73760 Ostfildern (DE)
(74) Vertreter: Heuckeroth, Volker, Dr.
(86) Internationale Anmeldenummer: EP9900453
(87) Internationale Veröffentlichungsnummer: WO9939623

(56) Entgegenhaltungen:
- WO-A-98/44376
- US-A- 4 756 304
- US-A- 4 969 450
- US-A- 5 359 992
- US-A- 5 706 143

## Beschreibung

Die Erfindung betrifft ein Endoskop, insbesondere Video-Endoskop, mit einem Endoskopschaft am distalen Ende und mit einem Fassungsanschluß am proximalen Ende zum Anschließen eines optischen Aufnahmegerätes, beispielsweise einer Videokamera, und mit einem Endoskopgehäuse zwischen dem Endoskopschaft und dem Fassungsanschluß, wobei das Endoskopgehäuse mit dem Fassungsanschluß direkt verbunden ist und der Fassungsanschluß über ein Drehgelenk relativ zum Endoskopschaft um die Längsachse des Endoskops verdrehbar ist, wobei ferner in dem Endoskopgehäuse eine optisch abbildende Anordnung aufgenommen ist, die zur Fokussierung der Bildübertragung über eine Stelleinrichtung zumindest teilweise lageverstellbar ist.

Ein derartiges Endoskop ist aus der US-A-4 969 450 bekannt.

Im Bereich der Endoskopie gewinnen immer mehr solche endoskopischen Systeme an Bedeutung, bei denen das durch das Endoskop beobachtbare Inspektions- bzw. Operationsgebiet im menschlichen Körper vom Operateur nicht direkt durch das Okular des Endoskops beobachtet wird, sondern über ein an das Endoskop angeschlossenes optisches Aufnahmegerät, beispielsweise eine Videokamera, auf einem optischen Wiedergabegerät, beispielsweise einem Bildschirm, sichtbar gemacht wird.

Die für einen derartigen Einsatz vorgesehenen Endoskope weisen dazu an ihrem proximalen Ende einen Fassungsanschluß zum Anschließen des Aufnahmegerätes auf.

Dabei sind derzeit verschiedene Typen von Fassungsanschlüssen im Gebrauch, und zwar Bajonett- oder Schraubfassungsanschlüsse. Diese Fassungsanschlüsse unterliegen Normen, um eine hohe Kompatibilität zwischen den erhältlichen objektivseitigen Fassungsanschlüssen und den entsprechenden Fassungsanschlüssen der Aufnahmegeräte zu gewährleisten.

Gemäß einer DIN-Norm ist beispielsweise bei einem als Fassungsanschluß C oder auch als C-Mount bezeichneten Fassungsanschluß des Kameraobjektivs das Auflagemaß, d.h. der Abstand der kameraseitigen Bildebene von der Auflagefläche des Kameragehäuses an dem objektivseitigen Fassungsanschluß definiert vorgegeben.

Da die Bildebene somit in der Kamera festliegt, muß eine Fokussierung des Strahlengangs im Objektiv erfolgen.

Wird ein derartiger Fassungsanschluß C bei einem Endoskop für die Video-Endoskopie verwendet, ist es daher erforderlich, die Fokussierung, d.h. die Scharfstellung der optischen Abbildung, in das Endoskop zu integrieren. Dazu ist zwischen dem Endoskopschaft und dem Fassungsanschluß ein Endoskopgehäuse angeordnet, in dem eine optisch abbildende Anordnung, in der Regel ein Linsensystem, aufgenommen ist. Zur Fokussierung ist die optisch abbildende Anordnung über eine von dem Operateur bedienbare Stelleinrichtung lageverstellbar. Die vorliegende Erfindung ist jedoch nicht auf einen derartigen Fassungsanschluß beschränkt.

Weiterhin ist es bei solchen Endoskopen für die Video-Endoskopie erforderlich, daß die an das Endoskop angeschlossene Videokamera relativ zum Endoskopschaft um die Längsachse des Endoskops verdrehbar ist, um bei einer Seitenblickoptik einen möglichst großen Bereich des Operationsgebietes optisch erfassen zu können und verschiedene Bildlagen auf dem Wiedergabegerät zu haben. Da die Videokamera mit dem Fassungsanschluß drehfest verbunden wird, bedeutet dies, daß der Fassungsanschluß relativ zum Endoskopschaft verdrehbar ausgebildet sein muß.

Bei dem aus der eingangs genannten US-A-4 969 450 bekannten Endoskop ist das dafür erforderliche Drehgelenk dadurch realisiert, daß das Endoskopgehäuse zwischen Schaft und Fassungsanschluß zweiteilig ausgebildet ist, d.h. eine Unterbrechung aufweist, wobei der Fassungsanschluß drehfest mit dem einen Gehäuseteil und der Endoskopschaft drehfest mit dem anderen Gehäuseteil verbunden ist.

An der Verbindungsstelle der beiden Gehäuseteile ist das Endoskopgehäuse somit offen, wobei eine Abdichtung des die optisch abbildende Anordnung enthaltenden Innenraums des Endoskopgehäuses bei dem bekannten Endoskop durch einen O-Ring zwischen den beiden Gehäuseteilen bewerkstelligt ist.

Eine derartige Ausgestaltung des eingangs genannten Endoskops ist jedoch nachteilig, weil die O-Ring-Dichtung nicht auf Dauer den Bedingungen der Sterilisation in einem Autoklaven standhalten kann. In einem Autoklaven wird das Endoskop im gespannten und gesättigten Wasserdampf bei über 120°C sterilisiert. Bei diesen Bedingungen kann es auch bei geeigneter Materialwahl des O-Rings zu Verschleißerscheinungen des O-Rings kommen, so daß bei mehrmaligen Sterilisationen in dem Autoklaven dann die Dichtwirkung nachläßt und Feuchtigkeit in den Innenraum des Endoskopgehäuses gelangen kann, die zur Beeinträchtigung der optisch abbildenden Anordnung in dem Endoskopgehäuse, beispielsweise durch Beschlagen der Linsen, und somit zur Unbrauchbarkeit des Endoskops führt oder zumindest einen erhöhten Wartungsaufwand zur Folge hat.

Das Dokument US-A-5 359 992 offenbart eine Kupplung zum Verbinden eines Endoskops mit einer Videokamera, wobei die Kupplung einen Fassungsanschluß zum Anschließen der Videokamera aufweist. Die Kupplung und damit der Fassungsanschluß sind demnach nicht Teil des Endoskops selbst. Der Fassungsanschluß ist mit dem Gehäuse der Kupplung verschraubt und dementsprechend nicht relativ zum Gehäuse der Kupplung verdrehbar. Innerhalb des Gehäuses der Kupplung ist eine optisch abbildende Anordnung aufgenommen, die zur Fokussierung der Bildübertragung über eine durch das Gehäuse hindurch wirkende magnetische Kupplung lageverstellbar ist.

Des weiteren ist aus dem Dokument US-A-4 756 304 ein arthroskopisches Videokamerasystem bekannt, das wie bei dem zuvor genannten Dokument eine Kupplung umfaßt, mit der eine Videokamera mit einem Arthroskop, d.h. einem Endoskop, verbunden werden kann. Die Kupplung weist am proximalen Ende einen Anschluß zum Anschließen der Videokamera und am distalen Ende einen Anschluß zum Anschließen des Endoskops auf, wobei der Anschluß zum Anschließen des Endoskops einen Stellring umfaßt, über den das Endoskop relativ zur Kupplung durch Drehen des Stellrings verdreht werden kann.

Der Erfindung liegt die Aufgabe zugrunde, ein Endoskop der eingangs genannten Art dahingehend weiterzubilden, daß das Endoskop auf Dauer den Bedingungen in einem Autoklaven standhalten kann.

Erfindungsgemäß wird diese Aufgabe hinsichtlich des eingangs genannten Endoskops dadurch gelöst, daß das Endoskopgehäuse durchgehend hermetisch geschlossen ausgebildet ist, und daß das Drehgelenk außerhalb des Endoskopgehäuses und am Fassungsanschluß ausgebildet ist.

Im Unterschied zu der geteilten Ausgestaltung des Endoskopgehäuses des zuvor erwähnten bekannten Endoskops ist bei dem erfindungsgemäßen Endoskop das Endoskopgehäuse hermetisch geschlossen ausgebildet. Hermetisch geschlossen bedeutet, daß keine Öffnungen oder durch Dichtungselemente abgedichtete Unterbrechungen, wie Gelenke, in dem Endoskopgehäuse vorgesehen sind. Der Innenraum des Endoskopgehäuses ist somit vollständig gegen den Außenraum gegen Kontaminationen hermetisch abgeschlossen, so daß auch bei einer Sterilisation mit Wasserdampf in einem Autoklaven keinerlei Feuchtigkeit in den Innenraum des Endoskopgehäuses gelangen kann. Das hermetisch geschlossene Endoskopgehäuse kann einstückig oder mehrstückig ausgebildet sein, wobei bei einer mehrstückigen Ausgestaltung die verschiedenen Stücke fest und dicht mit einander verbunden sind, beispielsweise durch Schweißen, Löten oder dergleichen, um die Bedingungen einer hermetischen Geschlossenheit des Endoskopgehäuses zu erfüllen. Das erfindungsgemäße Endoskop hält somit auf Dauer den Bedingungen in einem Autoklaven stand, weil keine Dichtungen wie O-Ringe verwendet werden, die auf Dauer ermüden können. Weiterhin ist es erfindungsgemäß vorgesehen, das zur Verdrehbarkeit des Fassungsanschlusses relativ zum Endoskopschaft erforderliche Drehgelenk außerhalb des geschlossenen Gehäuses auszubilden. Im Unterschied zu dem bekannten Endoskop ist das Drehgelenk somit nicht in das Endoskopgehäuse in Form einer zweiteiligen Ausgestaltung desselben integriert, wodurch der Vorteil erzielt wird, daß die Dichtigkeit des Endoskopgehäuses nicht durch das Drehgelenk beeinträchtigt wird.

Die der Erfindung zugrunde liegende Aufgabe wird somit vollkommen gelöst.

In einer weiteren Ausgestaltung ist das Drehgelenk im Bereich des proximalen Endes des Endoskopgehäuses angeordnet.

Dadurch, daß das Drehgelenk am proximalen Ende des Endoskopgehäuses angeordnet ist, wird der Vorteil erzielt, daß das im Endoskopschaft und Endoskopgehäuse angeordnete Bildübertragungssystem zueinander unverdrehbar ausgebildet werden kann, weil nur der Fassungsanschluß, der üblicherweise selbst kein Abbildungssystem enthält, drehbar ist. Dadurch werden auf vorteilhafte Weise durch Drehung der Bildübertragungssysteme relativ zueinander möglicherweise verursachte Abbildungsfehler vermieden.

In einer weiteren bevorzugten Ausgestaltung weist der Fassungsanschluß an seinem distalen Ende eine auf dem Endoskopschaft umfänglich angeordnete und drehbar gelagerte Hülse auf.

Durch diese Maßnahme wird auf mechanisch vorteilhaft einfache Weise ein außerhalb des geschlossenen Endoskopgehäuses ausgebildetes Drehgelenk geschaffen, das die Dichtigkeit des Endoskopgehäuses in keiner Weise beeinträchtigt und das vom Operateur leicht bedienbar ist, wobei zusätzlich der Vorteil geschaffen wird, daß das Endoskop axial kurzbauend ist, da die Hülse das Endoskopgehäuse axial teilweise umschließt.

In einer weiteren bevorzugten Ausgestaltung ist das Endoskopgehäuse drehfest mit dem Endoskopschaft verbunden.

Diese Maßnahme hat nun den Vorteil, daß das in dem Endoskopschaft enthaltene schaftfeste Bildübertragungssystem, bspw. ein Relaislinsensystem, bei einer Verdrehung der Kamera relativ zum Endoskopschaft gegenüber der in dem Endoskopgehäuse enthaltenen optisch abbildenden Anordnung nicht verdreht wird, sondern diese Bildübertragungssysteme stets die gleiche Drehlage zueinander aufweisen. Somit können Bildlageabweichungen, die von Toleranzen des in dem Endoskopschaft enthaltenen Bildübertragungssystemes und der in dem Endoskopgehäuse enthaltenen optisch abbildenden Anordnung herrühren und durch einmaliges Ausrichten dieser Systeme relativ zueinander bei der Montage des Endoskops korrigiert werden können, nicht erneut während der Verdrehung des Fassungsanschlusses relativ zu dem Endoskopschaft während des Einsatzes des Endoskops auftreten, so daß ein nachträgliches Justieren nicht mehr erforderlich ist. Bei dem eingangs genannten bekannten Endoskop ist dagegen der Endoskopschaft relativ zu dem Endoskopgehäuse und der darin enthaltenen optischen Anordnung verdrehbar, so daß es bei einer Verdrehung des Endoskopschaftes relativ zu dem Gehäuse zu Bildlageabweichungen und Abbildungsfehlern kommen kann.

In einer weiteren bevorzugten Ausgestaltung wirkt die Stelleinrichtung von außen durch das geschlossene Endoskopgehäuse auf die optisch abbildende Anordnung.

Durch diese Maßnahme werden im Unterschied zu dem eingangs genannten bekannten Endoskop weitere Öffnungen in dem Endoskopgehäuse, die entsprechend nach außen durch Dichtmittel abgedichtet werden müssen, auf vorteilhafte Weise vermieden. Bei dem bekannten Endoskop weist die Stelleinrichtung einen drehbaren Stellring auf, der mechanisch durch eine Öffnung in dem Endoskopgehäuse mit der optisch abbildenden Anordnung verbunden ist. Zur erforderlichen Abdichtung des Innenraums des Endoskopgehäuses sind distal- und proximalseitig an dem Stellring zwei O-Ringe vorgesehen, die wiederum für eine mehrmalige Sterilisation des Endoskops in einem Autoklaven ungeeignet sind. Bei dem erfindungsgemäßen Endoskop ist dagegen vorgesehen, das Endoskopgehäuse hermetisch geschlossen auszubilden und die Stelleinrichtung von außen auf die optisch abbildende Anordnung wirken zu lassen, wodurch Abdichtungen über O-Ringe oder dgl. vermieden werden, so daß die Autoklavierbarkeit des erfindungsgemäßen Endoskops weiter verbessert wird.

In einer weiteren bevorzugten Ausgestaltung wirkt die Stelleinrichtung über einen magnetischen Kraftschluß auf die optisch abbildende Anordnung.

Diese Maßnahme hat nun den Vorteil, daß der magnetische Kraftschluß eine Lageverstellung der optisch abbildenden Anordnung in dem Endoskopgehäuse von außen bewirken kann, ohne daß dazu Öffnungen in dem Endoskopgehäuse, wie bei einer mechanischen Verbindung, erforderlich sind. Dies bedeutet jedoch nicht zwangsläufig, daß das Endoskopgehäuse nicht aus Metall bestehen kann, denn es sind Metalle erhältlich, die den Magnetfluß nicht wesentlich beeinflussen. Das Endoskopgehäuse könnte jedoch ebenso aus einem nichtmetallischen Material, etwa Kunststoff oder Keramik, bestehen.

In einer weiteren bevorzugten Ausgestaltung weist die Stelleinrichtung einen Stellring auf, der um die Außenseite des Endoskopgehäuses angeordnet ist und zur Lageverstellung zumindest eines Teils der optisch abbildenden Anordnung relativ zum Endoskopschaft verdrehbar ist.

Bei dieser Ausgestaltung der Stelleinrichtung wird der Stellring relativ zum Endoskopschaft verdreht, um die optisch abbildende Anordnung zur Fokussierung des Strahlenganges zu verstellen. Bei losgelassenem Stellring dreht sich dieser durch eine ggf. vorgesehene entsprechende reibschlüssige Verbindung mit dem Endoskopschaft mit. Dies ist dann vorteilhaft, wenn der Endoskopschaft eine Seitenblickoptik aufweist und im Operationsgebiet gedreht wird, um einen größeren Bereich des Operationsgebiets zu erfassen.

In einer weiteren bevorzugten Ausgestaltung weist die Stelleinrichtung einen Stellring auf, der zur Lageverstellung zumindest eines Teils der optisch abbildenden Anordnung relativ zum Fassungsanschluß verdrehbar ist.

Diese Maßnahme hat nun den Vorteil, daß das Endoskop eine Einhand-Bedienung ermöglicht, indem der Operateur zur Fokussierung mit zwei Fingern der Hand, mit der er die Kamera hält und auch relativ zum Endoskopschaft drehen kann, den Stellring relativ zum Fassungsanschluß verdreht, ohne dabei den Endoskopschaft festhalten zu müssen.

In einer weiteren bevorzugten Ausgestaltung weist die Stelleinrichtung zumindest ein um die Außenseite des Endoskopgehäuses drehbar angeordnetes äußeres Ringelememt, das zumindest einen Magnet trägt, und zumindest ein innerhalb des Endoskopgehäuses angeordnetes drehbares inneres Ringelement auf, das ebenfalls zumindest einen Magnet trägt, wobei eine Drehung des äußeren Ringelementes das innere Ringelement bewegt und die Bewegung des inneren Ringelementes der Lageverstellung zumindest eines Teils der optisch abbildenden Anordnung dient. Die Magnete können auch als mehrpolig am inneren bzw. äußeren Umfang magnetisierte Ringmagnete ausgebildet sein.

Durch diese Maßnahme wird auf vorteilhafte Weise eine Stelleinrichtung in der Art einer Magnetkupplung realisiert, die von außen durch das geschlossene Endoskopgehäuse über einen magnetischen Kraftschluß auf die optisch abbildende Anordnung wirkt. Das äußere Ringelement ist dabei bevorzugt mit dem zuvor erwähnten Stellring zur Bedienung der Stelleinrichtung verbunden.

Dabei ist weiterhin bevorzugt, wenn die Bewegung des inneren Ringelementes über eine mechanische Verbindung in eine axiale Bewegung zumindest eines Teils der optisch abbildenden Anordnung umgesetzt wird.

Diese Maßnahme hat nun den Vorteil, daß die zuvor erwähnte Magnetkupplung axial sehr kleinbauend ausgestaltet werden kann, weil das innere und das äußere Ringelement nur eine Drehbewegung ausführen, während die Drehbewegung des inneren Ringelementes über die mechanische Verbindung, beispielsweise einen Gewindegang, die axiale Bewegung der optisch abbildenden Anordnung zur Fokussierung der Abbildung bewirkt.

In einer weiteren bevorzugten Ausgestaltung weist die Stelleinrichtung zwei um die Außenseite des Endoskopgehäuses angeordnete äußere Ringelemente, die jeweils zumindest einen Magnet tragen, und zwei innerhalb des Endoskopgehäuses angeordnete innere Ringelemente auf, die ebenfalls jeweils zumindest einen Magnet tragen, wobei eine Drehbewegung der äußeren Ringelemente das jeweilige zugehörige innere Ringelement bewegt, und wobei eine Drehung des einen äußeren Ringelementes relativ zu dem anderen äußeren Ringelement eine Lageverstellung zumindest eines Teils der optisch abbildenden Anordnung bewirkt.

Diese Ausgestaltung der Stelleinrichtung mit zwei relativ zueinander verdrehbaren Magnetkupplungen eröffnet in Verbindung mit dem zuvor erwähnten Stellring, der zur Lageverstellung der optisch abbildenden Anordnung relativ zu dem Fassungsanschluß verdrehbar ist, eine vorteilhafte Ausgestaltung des erfindungsgemäßen Endoskops für eine Einhandbedienung.

Dabei ist es bevorzugt, wenn das eine äußere Ringelement drehfest mit dem Fassungsanschluß verbunden ist und das andere äußere Ringelement relativ zu diesem verdrehbar ist.

Diese Maßnahme hat den Vorteil, daß zur Relativbewegung der beiden Magnetkupplungen nur das eine äußere Ringelement über einen Stellring zur Fokussierung verdreht werden muß, während das andere Ringelement drehfest mit dem Fassungsanschluß verbunden ist. Wird das relativ zu dem Fassungsanschluß verdrehbare äußere Ringelement nicht verdreht, dreht sich dieses bei einer Drehung des Fassungsanschlusses relativ zu dem Endoskopschaft mit, so daß dann keine Fokussierung erfolgt, wenn dies nicht erwünscht ist.

Dabei ist es bevorzugt, wenn das nicht drehfest mit dem Fassungsanschluß verbundene äußere Ringelement mit dem Fassungsanschluß reibschlüssig verbunden ist.

Durch diese Maßnahme wird der Vorteil erzielt, daß sich das relativ zu dem Fassungsanschluß verdrehbare äußere Ringelement mit diesem mitdreht, wenn der Fassungsanschluß bei losgelassenem Stellring relativ zum Endoskopschaft verdreht wird, ohne daß dabei eine Fokussierung erfolgt. Die erwähnte reibschlüssige Verbindung kann bspw. durch einen an dem äußeren Ringelement vorgesehenen oder an einem mit dem äußeren Ringelement verbundenen Stellring vorgesehenen O-Ring bewerkstelligt werden.

In einer weiteren bevorzugten Ausgestaltung umfaßt die optisch abbildende Anordnung eine axial verschiebbare Okularlinsenanordnung des Endoskops und eine ortsfeste Objektivlinsenanordnung des optischen Aufnahmegerätes, und wird mittels der Stelleinrichtung zur Fokussierung die Okularlinsenanordnung verschoben.

Diese Maßnahme hat den Vorteil, daß zur Fokussierung ein geringerer Stellweg der optischen Anordnung erforderlich ist, da die Okularlinsenanordnung eine geringere Brennweite als die Objektivlinsenanordnung aufweist. Ein weiterer Vorteil besteht darin, daß im Fall, daß die Okularlinsenanordnung eine in der Zwischenbildebene des Okulars angeordnete Blende aufweist, die mit der Okularlinsenanordnung gemeinsam verschiebbar ist, diese Blende stets scharf abgebildet wird. Dies eröffnet die Möglichkeit, an der Blende eine Markierung anzubringen, an Hand derer die Drehstellung der Kamera relativ zu dem bezüglich der Blende drehfesten Endoskopschaft bestimmt werden kann.

Weiterhin ist es bevorzugt, wenn die optisch abbildende Anordnung drehfest in dem Endoskopgehäuse angeordnet ist.

Diese Maßnahme hat nun in Verbindung mit einem drehfest an dem Endoskopgehäuse befestigten Endoskopschaft den Vorteil, daß das Bildübertragungssystem des Endoskopschafts stets die gleiche Drehstellung bezüglich der optisch abbildenden Anordnung aufweist, d.h. diese nicht gegeneinander verdreht werden können, so daß Bildlageabweichungen infolge einer Verdrehung dieser beiden Baugruppen relativ zueinander vermieden werden.

In einer weiteren bevorzugten Ausgestaltung ist in dem Endoskopgehäuse eine relativ zu diesem drehfeste Blende angeordnet, die eine Markierung zur Bestimmung der Drehlage des Fassungsanschlusses relativ zum Endoskopschaft aufweist.

Diese Maßnahme hat, wie zuvor erwähnt, den Vorteil, daß die Drehstellung des Fassungsanschlusses relativ zum Endoskopschaft beispielsweise auf dem Bildschirm angezeigt und beobachtet werden kann.

Dabei ist es bevorzugt und vorteilhaft, wenn die Blende in einer Zwischenbildebene angeordnet ist und zusammen mit der Okularlinsenanordnung verschoben wird.

Weitere Vorteile ergeben sich aus der nachfolgenden Beschreibung der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach näher beschrieben. Es zeigen:
- Fig. 1: einen Längsschnitt durch ein Endoskop gemäß einem ersten Ausführungsbeispiel der Erfindung; und
- Fig. 2: einen teilweisen Längsschnitt durch ein Endoskop gemäß einem zweiten Ausführungsbeispiel der Erfindung.

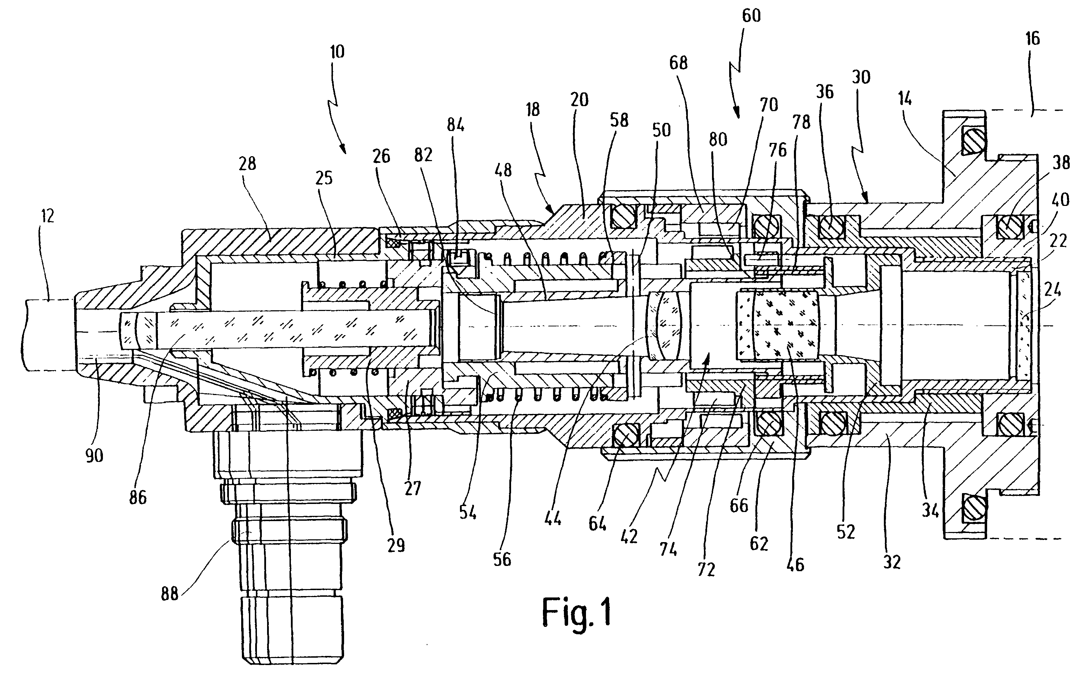

In Fig. 1 ist ein mit dem allgemeinen Bezugszeichen 10 versehenes Endoskop dargestellt. Das Endoskop 10 ist ein Video-Endoskop, bspw. zur Verwendung in der Arthroskopie.

An seinem distalen Ende weist das Endoskop 10 einen durch unterbrochene Linien angedeuteten Endoskopschaft 12 auf. An seinem proximalen Ende weist das Endoskop 10 einen Fassungsanschluß 14 zum Anschließen eines optischen Aufnahmegeräts 16, bspw. einer Videokamera, auf, das in Fig. 1 durch unterbrochene Linien angedeutet ist.

Der Fassungsanschluß 14 ist ein der DIN 15 735 (Teil 2) entsprechender Fassungsanschluß C, der auch als C-Mount bezeichnet wird.

Zwischen dem Endoskopschaft 12 und dem Fassungsanschluß 14 weist das Endoskop 10 ein Endoskopgehäuse 18 auf, dessen Innenraum nach außen hermetisch geschlossen ist.

Das Endoskopgehäuse 18 ist drehfest mit dem Endoskopschaft 12 verbunden.

Das Endoskopgehäuse 18 wird durch ein im wesentlichen zylindrisches Gehäusehauptteil 20 gebildet, das an seinem proximalen Ende 22 durch ein Deckglas 24 hermetisch geschlossen ist. Das Deckglas 24 ist beispielsweise in die Öffnung des Gehäusehauptteils 20 am proximalen Ende 22 mittels eines feuchtigkeits- und hitzebeständigen Klebstoffs eingeklebt bzw. eingelötet.

Am distalen Ende 26 des Gehäusehauptteils 20 ist dieses drehfest und hermetisch dicht über eine verklebte und zusätzlich abgedichtete Verschraubung mit einer Hülse 25 verbunden, die sich wiederum drehfest und hermetisch dicht an das in Fig. 1 nicht dargestellte, die Linsenanordnung 86 umschließende, Innenrohr des drehfesten Endoskopschafts 12 anschließt, beispielsweise damit verlötet ist. Das Gehäusehauptteil 20 könnte auch einstückig bis zu dem Endoskopschaft 12 durchgehend ausgebildet sein. Weitere Elemente 27, 28 und 29 sind im distalen Bereich des Endoskopgehäuses 18 angeordnet.

Der Fassungsanschluß 14 ist über ein Drehgelenk 30 relativ zu dem Endoskopschaft 12 um die Längsachse des Endoskops 10 verdrehbar. Zur Ausbildung des Drehgelenks 30 ist der Fassungsanschluß 14 drehbar an der Außenseite des Endoskopgehäuses 18 befestigt. Der Fassungsanschluß 14 weist eine Hülse 32 auf, die das Endoskopgehäuse 18 bzw. das Gehäusehauptteil 20 am proximalen Ende zumindest teilweise umfänglich umgibt. Die Hülse 32 ist drehbar auf einer fest mit dem Gehäusehauptteil 20 verbundenen inneren Hülse 34 gelagert. Damit der Fassungsanschluß 14 gegenüber dem Endoskopschaft 12 nur bei Ausübung eines definierten Mindest-Drehmomentes verdrehbar ist, ist der Fassungsanschluß 14 auf dem Endoskopgehäuse 18 reibschlüssig gelagert. Dazu ist ein in der inneren Hülse 34 distalseitig aufgenommener O-Ring und ein in einem Ring 40 aufgenommener O-Ring am proximalen Ende des Fassungsanschlusses 14 vorgesehen.

In dem Endoskopgehäuse 18 ist eine optisch abbildende Anordnung 42 angeordnet. Die optisch abbildende Anordnung 42 umfaßt eine Okularlinsenanordnung 44 des Endoskops 10 und eine Objektivlinsenanordnung 46 des optischen Aufnahmegerätes 16.

Die Okularlinsenanordnung 44 ist in einer Okularfassung 48 unbeweglich befestigt. Die Okularfassung 48 ist in dem Endoskopgehäuse 18 über einen Verdrehsicherungsstift 50 relativ zu dem Endoskopgehäuse 18 und somit zu dem Endoskopschaft 12 drehfest angeordnet. Damit ist auch die Okularlinsenanordnung 44 drehfest bzgl. des Endoskopschafts 12.

Die Objektivlinsenanordnung 46 ist in dem Endoskopgehäuse 18 in einer bezüglich des Endoskopgehäuses 18 lagefesten Objektivfassung 52 befestigt.

Die Okularfassung 48 ist in einer lagefesten Okularführung 54 axial verschiebbar aufgenommen. Um die Okularführung 54 herum ist eine Druckfeder 56 angeordnet, die über einen relativ zu der Okularführung 54 axial verschiebbaren Ring 58 gegen den fest mit der Okularfassung 48 verbundenen Verdrehsicherungsstift 50 drückt und die Okularfassung 48 dadurch in Richtung zum proximalen Ende des Endoskops 10 vorspannt. Die Okularfassung 48 ist somit axial verschiebbar, während die Objektivfassung 52 axial unverschiebbar und drehfest ist.

Ferner weist das Endoskop 10 eine Stelleinrichtung 60 zur Fokussierung des von dem Endoskopschaft 12 zu dem optischen Aufnahmegerät 16 verlaufenden Strahlenganges auf, d.h. zur Scharfstellung des von dem optischen Aufnahmegerät 16 empfangenen Bildes. Wie sich aus der folgenden Beschreibung ergibt, wirkt die Stelleinrichtung 60 von außen durch das geschlossene Gehäuse 18 hindurch auf die optisch abbildende Anordnung 42, um diese zur Fokussierung lagezuverstellen.

Die Stelleinrichtung 60 weist einen Stellring 62 auf, der die Außenseite des Endoskopgehäuses 18 umgibt und relativ zu diesem verdrehbar ist. Der Stellring 62 ist über einen O-Ring 64 und einen O-Ring 66 reibschlüssig auf dem Gehäusehauptteil 20 gelagert, so daß zur Drehung des Stellrings 62 auf dem Gehäusehauptteil 20 ein Drehmoment ausgeübt werden muß, das den durch die O-Ringe 64 und 66 definierten Reibschluß überwindet.

Die Stelleinrichtung 60 weist ferner ein um die Außenseite des Endoskopgehäuses 18 angeordnetes äußeres Ringelement 68 auf. Das äußere Ringelement 68 ist umfänglich geschlossen und trägt auf seiner Innenseite umfänglich verteilt Magnete 70, und zwar 24 derartiger Magnete 70. Das äußere Ringelement 68 bildet einen magnetischen Rückschluß zwischen den einzelnen Magneten 70, d.h. es treten keine Feldlinien aus der Außenseite des äußeren Ringelements 68 aus. Das äußere Ringelement 68 ist außerdem mit seiner Außenseite fest mit dem Stellring 62 verbunden.

Innerhalb des Endoskopgehäuses 18 ist ein inneres Ringelement 72 dem äußeren Ringelement 68 gegenüberliegend angeordnet, das den Magneten 70 gegenüberliegende Magnete 74 in gleicher Anzahl trägt. Eine Drehung des äußeren Ringelements 68 durch Drehen des Stellrings 62 bewirkt eine Drehung des inneren Ringelements 72 in gleicher Drehrichtung. Der magnetische Kraftschluß zwischen den Magneten 70 und 74 wirkt durch das Gehäuse 18, genauer gesagt durch das Gehäusehauptteil 20 hindurch, wobei das Gehäusehauptteil 20 dazu aus einem geeigneten Material besteht.

. Zur Übertragung der Drehbewegung des inneren Ringelements 72 in eine axiale Verschiebung der Okularfassung 48 ist das innere Ringelement 72 über einen Mitnehmerstift 76 mit einer Mutter 78 drehfest verbunden, wobei die Mutter 78 wiederum über ein auf ihrer Innenseite ausgebildetes Gewinde mit einem entsprechenden Gewinde am proximalen Ende 80 der Okularfassung 48 in Verbindung steht. Eine Drehbewegung des inneren Ringelements 72 bewirkt somit, daß die durch den Verdrehsicherungsstift 50 undrehbare Okularfassung 48 über die Gewindeverbindung am distalen Ende 80 durch die Mutter 78 axial in der Okularführung 54 verschoben wird.

In der Okularfassung 48 ist ferner eine Blende 82 fest montiert und daher zusammen mit der Okularlinsenanordnung 44 gemeinsam axial verschiebbar. Ferner sind für die Okularführung 54 drei Justierschrauben 84 vorgesehen, von denen in Fig. 1 nur eine dargestellt ist, mittels der die Baugruppe aus der Okularlinsenanordnung 44 und der Blende 82 gegenüber dem Endoskopschaft 12 in radialer Richtung justiert werden kann, um Bildlageabweichungen bei der Bildübertragung zu korrigieren.

Ferner ist in dem Endoskopgehäuse 18 eine weitere Linsenanordnung 86 als Bestandteil des Bildübertragungssystems des Endoskopschafts 12 aufgenommen, die bzgl. der Blende 82, der Okularlinsenanordnung 44 und der Objektivlinsenanordnung 46 drehfest ist.

Ferner weist das Endoskop 10 einen Anschluß 88 zum Anschließen eines Lichtleitersystems auf, von dem Lichtleiter 90 in den Endoskopschaft 12 zur Ausleuchtung des Inspektionsgebiets führen.

Im folgenden wird nun die Funktionsweise des Endoskops 10 beschrieben. Der Fassungsanschluß 14 und damit das an diesem angeschlossene optische Aufnahmegerät 16 kann relativ zu dem Endoskopschaft 12 und dem damit drehfest verbundenen Endoskopgehäuse 18 verdreht werden. Zum Fokussieren wird der Stellring 62 gedreht, wobei das Endoskopgehäuse 18 dazu festgehalten wird. Eine Drehung des Stellrings 62 führt über die damit verbundene Drehung des äußeren Ringelements 68 zu einer Drehung des inneren Ringelements 72 in gleicher Drehrichtung, wodurch die Okularlinsenanordnung 44 durch die mechanische Gewindeverbindung zwischen dem inneren Ringelement 72, dem Mitnehmerstift 76, der Mutter 78 und dem proximalen Ende 80 der Okularfassung 48 relativ zu der Objektivlinsenanordnung 46 axial verschoben wird.

An der Blende 82 ist noch eine in Fig. 1 nicht dargestellte Markierung an ihrem Umfang angebracht, die es ermöglicht, die Drehstellung des Fassungsanschlusses 14 und damit des optischen Aufnahmegeräts 16 relativ zu dem Endoskopschaft 12, der bezüglich der Blende 82 drehfest ist, zu bestimmen. Die Linsenanordnung 86, die Blende 82, die Okularlinsenanordnung 44 und die Objektivlinsenanordnung 46 weisen unabhängig von der Drehstellung des Fassungsanschlusses 14 relativ zu dem Endoskopschaft 12 stets die gleiche Drehorientierung zueinander auf.

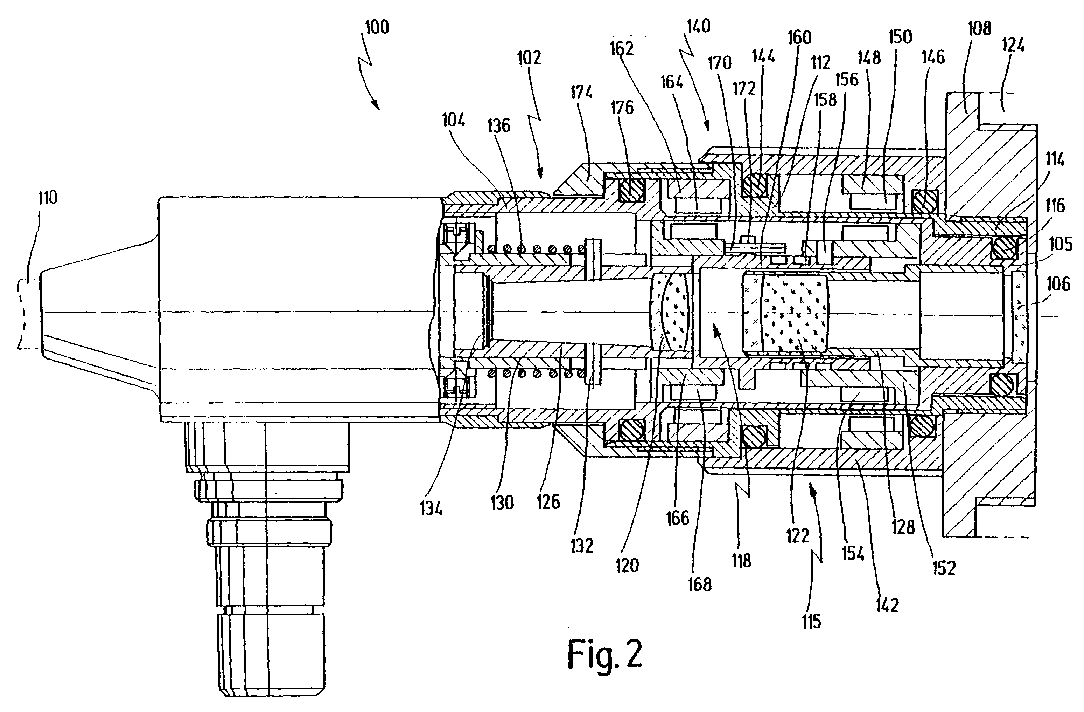

In Fig. 2 ist ein weiteres Ausführungsbeispiel eines mit dem Bezugszeichen 100 versehenen Endoskops dargestellt. Soweit sich aus der nachfolgenden Beschreibung nichts anderes ergibt, entspricht das Endoskop 100 hinsichtlich Konstruktion und Funktion dem Endoskop 10 in Fig. 1.

Das Endoskop 100 weist ein Endoskopgehäuse 102 auf, das aus einem Gehäusehauptteil 104 gebildet wird, das am proximalen Ende wiederum durch ein Deckglas 106 hermetisch dicht verschlossen ist.

Ein Fassungsanschluß 108 am proximalen Ende des Endoskops 100 ist relativ zu einem Endoskopschaft 110, der mit dem Endoskopgehäuse 102 fest verbunden ist, verdrehbar auf dem Endoskopgehäuse 102 gelagert. Der Fassungsanschluß 108 ist fest mit einem hülsenförmigen Verbindungsteil 112 verbunden, wobei der Fassungsanschluß 108 über das proximale Ende 114 des Verbindungsteils 112 zur Ausbildung eines Drehgelenks 115 drehbar auf dem proximalen Ende 105 des Gehäusehauptteils 104 gelagert ist. O-Ringe 116 und 176 sorgen für ein definiertes Drehmoment bei der Verdrehung des Fassungsanschlusses 108 bezüglich des Endoskopgehäuses 102.

In dem Endoskopgehäuse 102 ist eine optisch abbildende Anordnung 118 aufgenommen, die eine Okularlinsenanordnung 120 des Endoskops 100 und eine Objektivlinsenanordnung 122 eines an dem Fassungsanschluß 108 anschließbaren optischen Aufnahmegeräts 124 umfaßt.

Die Okularlinsenanordnung 120 ist in einer Okularfassung 126 fest montiert, während die Objektivlinsenanordnung 122 in einer Objektivfassung 128 fest montiert ist. Die Objektivfassung 128 ist drehfest und axial unverschiebbar in dem Endoskopgehäuse 102 aufgenommen.

Die Okularfassung 126 ist dagegen in einer Okularführung 130 axial verschiebbar aufgenommen, jedoch durch einen Verdrehsicherungsstift 132 drehfest in dem Endoskopgehäuse 102 angeordnet. Ebenso ist eine Blende 134 in der Okularfassung 126 fest montiert. Eine Druckfeder 136 ist um die Okularführung 130 herum angeordnet und spannt die Okularfassung 126 über den Verdrehsicherungsstift 132 zum proximalen Ende des Endoskops 100 hin vor.

Das Endoskop 100 weist eine Stelleinrichtung 140 zur Fokussierung des vom Endoskopschaft 110 in das optische Aufnahmegerät 124 übertragenen Bildes auf.

Die Stelleinrichtung 140 weist einen Stellring 142 auf. Der Stellring 142 ist zur Lageverstellung der optisch abbildenden Anordnung 118 relativ zu dem Fassungsanschluß 108 verdrehbar. Dazu ist der Stellring 142 um das fest mit dem Fassungsanschluß 108 verbundene Verbindungsteil 112 herum drehbar gelagert. Ein von dem Verbindungsteil 112 getragener O-Ring 144 und ein von dem Stellring 142 getragener O-Ring 146 bewirken eine reibschlüssige Verbindung zwischen dem Stellring 142 und dem Verbindungsteil 112 und über diesen mit dem Fassungsanschluß 108, so daß der Stellring 142 nur bei Aufwendung eines bestimmten Drehmoments gegenüber dem Fassungsanschluß 108 verdreht werden kann. Der Stellring 142 ist selbstverständlich auch gegenüber dem Endoskopgehäuse 102 verdrehbar.

Die Stelleinrichtung 140 weist ein erstes äußeres Ringelement 148 auf, das auf der Außenseite des Endoskopgehäuses 102 und des Verbindungsteils 112 umfänglich angeordnet ist. Das erste äußere Ringelement 148 trägt auf seiner Innenseite, d.h. der dem Verbindungsteil 112 zugewandten Seite, Magnete 150, die auf der Innenseite des ersten äußeren Ringelements 148 umfänglich verteilt angeordnet sind.

Die Außenseite des äußeren Ringelements 148 ist fest mit dem Stellring 142 auf dessen Innenseite verbunden.

Dem ersten äußeren Ringelement 148 gegenüberliegend ist innerhalb des Gehäusehauptteils 104 ein erstes inneres Ringelement 152 angeordnet, das auf seiner Außenseite Magnete 154 trägt, die den Magneten 150 radial gegenüberstehen. Die Magnete 150 und 154 wirken durch das Verbindungsteil 112 und das Gehäusehauptteil 104 hindurch über einen magnetischen Kraftschluß zusammen.

An dem ersten inneren Ringelement 152 ist ein Stift 156 befestigt, der in eine wendelartige Nut 158 eingreift, die in einem Schieber 160 ausgebildet ist, der um die Objektivfassung 128 drehbar gelagert ist. Die Okularfassung 126 stützt sich dabei mit ihrem proximalen Ende gegen das distale Ende des Schiebers 160 ab. Die Druckfeder 136 spannt die Okularfassung 126 gegen den Schieber 160 vor.

Der Schieber 160 ist gegenüber der Objektivfassung 128 sowohl drehbar als auch axial verschiebbar gelagert. Das erste innere Ringelement 152 ist in dem Endoskopgehäuse 102 drehbar, jedoch axial feststehend angeordnet.

Die Stelleinrichtung 140 weist ein zweites äußeres Ringelement 162 auf, das um das Endoskopgehäuse 102 umfänglich auf dessen Außenseite angeordnet und relativ zu diesem um die Längsachse des Endoskops 100 drehbar ist. Das zweite äußere Ringelement 162 ist fest mit dem distalen Ende des Verbindungsteils 112 verbunden, das, wie bereits erwähnt, drehfest mit dem Fassungsanschluß 108 verbunden ist. Die Baugruppe aus dem zweiten äußeren Ringelement 162, dem Verbindungsteil 112 und dem Fassungsanschluß 108 bildet somit eine fest miteinander verbundene Einheit, die gegenüber dem Endoskopgehäuse 102 verdrehbar ist.

Das zweite äußere Ringelement 162 trägt auf seiner Innenseite wiederum umfänglich verteilt angeordnete Magnete 164. Auf der Innenseite des Endoskopgehäuses 102 ist ein zweites inneres Ringelement 166 angeordnet, das dem zweiten äußeren Ringelement 162 radial gegenüberliegt und in dem Innenraum des Endoskopgehäuses 102 drehbar gelagert ist. Das zweite innere Ringelement 166 trägt auf seiner Außenseite den Magneten 164 radial gegenüberstehende Magnete 168, die mit den Magneten 164 zusammenwirken. Zusammenwirken bedeutet wiederum, daß eine Drehung des zweiten äußeren Ringelements 162 eine gleichsinnige Drehung des zweiten inneren Ringelements 166 bewirkt.

Das zweite innere Ringelement 166 ist in dem Endoskopgehäuse 102 axial unverschiebbar aufgenommen. Das zweite innere Ringelement 166 umgibt teilweise den Schieber 160, wobei der Schieber 160 relativ zu dem zweiten inneren Ringelement 166 axial verschiebbar ist.

Ein mit dem zweiten inneren Ringelement 166 fest verbundener Mitnehmerstift 170 wirkt mit einer Nut 172, die an dem Schieber 160 ausgebildet ist, so zusammen, daß sich das zweite innere Ringelement 166 und der Schieber 160 nur gemeinsam in dem Endoskopgehäuse 102 drehen können, und zwar in beiden Drehrichtungen. Das innere Ringelement 166 und der Schieber 160 sind somit relativ zueinander unverdrehbar.

Ferner ist mit dem Verbindungsteil 112 an dessen distalem Ende eine Lagerhülse 174 fest verbunden, die sich somit mit dem Verbindungsteil 112 und dem Fassungsanschluß 108 gemeinsam um das Endoskopgehäuse 102 um die Längsachse des Endoskops 100 drehen kann. Über einen O-Ring 176 wird zusammen mit dem O-Ring 116 ein Mindest-Drehmoment zur Verdrehung des Fassungsanschlusses 108 und damit des optischen Aufnahmegeräts 124 relativ zu dem Endoskopgehäuse 102 und damit zu dem Endoskopschaft 110 durch reibschlüssige Verbindung definiert.

Im folgenden wird nun die Funktionsweise des Endoskops 100 erläutert. Dabei wird das Endoskopgehäuse 102 mit dem Endoskopschaft 110 als raumfestes Bezugssystem betrachtet.

Wird der Fassungsanschluß 108 in einer Hand festgehalten, so kann mit Daumen und Zeige- bzw. Mittelfinger der Stellring 142 zur Fokussierung bzw. zur Lageverstellung der optisch abbildenden Anordnung 118 relativ zu dem Fassungsanschluß 108 bzw. zu dem optischen Aufnahmegerät 124 verdreht werden. Eine Drehung des Stellrings 142 bewirkt eine Drehung des ersten äußeren Ringelements 148 und der mit diesem fest verbundenen Magnete 150. Die mit den Magneten 154 des ersten inneren Ringelements 152 durch das Verbindungsteil 112 und das Gehäusehauptteil 104 magnetisch zusammenwirkenden Magnete 150 des ersten äußeren Ringelements 148 bewirken eine zum ersten äußeren Ringelement 148 gleichsinnige Drehbewegung des ersten inneren Ringelements 152.

Da der Fassungsanschluß 108 festgehalten wird, steht das zweite äußere Ringelement 162 und damit auch das mit diesem magnetisch gekoppelte zweite innere Ringelement 166 still. Das erste äußere Ringelement 148 und das erste innere Ringelement 152 führen somit eine Relativdrehung zu dem zweiten äußeren Ringelement 162 bzw. dem zweiten inneren Ringelement 166 aus. Mit dem zweiten inneren Ringelement 166 wird auch der Schieber 160 durch den Mitnehmerstift 170 drehfest gehalten, so daß die Drehbewegung des ersten inneren Ringelements 152 bewirkt, daß der Stift 156 in der wendelartigen Nut 158 umläuft und dabei den Schieber 160 in Richtung proximales Ende des Endoskops 100 verschiebt. Da die Druckfeder 136 die Okularfassung 126 gegen den Schieber 160 drückt, folgt die Okularfassung 126 der axialen Bewegung des Schiebers 160. Bei einer Drehung des Stellrings 142 in entgegengesetzter Drehrichtung wird die Okularlinsenanordnung 120 entsprechend zum distalen Ende des Endoskops 100 gegen die Kraft der Druckfeder hin verschoben. Somit kann durch Drehen des Stellrings 142 bei festgehaltenem Fassungsanschluß 108 bzw. festgehaltenem optischen Aufnahmegerät 124 das Bild fokussiert, d.h. scharfgestellt werden.

Soll andererseits lediglich die Drehstellung des optischen Aufnahmegeräts 124 relativ zu dem Endoskopschaft 110 verändert werden, ohne daß eine Fokussierung durchgeführt werden soll, wird nur der Fassungsanschluß 108 bei losgelassenem Stellring 142 gedreht. In diesem Fall dreht sich das zweite äußere Ringelement 162 und über die magnetische Kopplung mit diesem das zweite innere Ringelement 166, das über den Mitnehmerstift 170 den Schieber 160 ebenfalls in Drehung versetzt. Da der Stellring 142 mit dem Fassungsanschluß 108 über die O-Ringe 144 und 146 reibschlüssig verbunden ist, dreht sich auch der Stellring 142 mit dem Fassungsanschluß 108 mit. Damit dreht sich auch das erste äußere Ringelement 148 und das mit diesem magnetisch gekoppelte erste innere Ringelement 152 mit. Das erste äußere Ringelement 148 und das zweite äußere Ringelement 162 führen somit keine Relativdrehung zueinander aus, sondern drehen sich gemeinsam um das Endoskopgehäuse 102. Somit dreht sich der Stift 156 an dem ersten inneren Ringelement 152 gemeinsam mit dem durch den Mitnehmerstift 170 vom zweiten inneren Ringelement 166 drehangetriebenen Schieber 160 mit, so daß der Stift 156 nicht in der wendelartigen Nut 158 umläuft. Somit tritt keine axiale Verschiebung der Okularfassung 126 und damit der Okularlinsenanordnung 120 auf.

## Patentansprüche

1. Endoskop, insbesondere Video-Endoskop, mit einem Endoskopschaft (12; 110) am distalen Ende und mit einem Fassungsanschluß (14;108) am proximalen Ende zum Anschließen eines optischen Aufnahmegerätes (16; 124), bspw. einer Videokamera, und mit einem Endoskopgehäuse (18; 102) zwischen dem Endoskopschaft (12; 110) und dem Fassungsanschluß (14; 108), wobei das Endoskopgehäuse (18; 102) mit dem Fassungsanschluß (14; 108) direkt verbunden ist und der Fassungsanschluß (14; 108) über ein Drehgelenk (30; 115) relativ zum Endoskopschaft (12; 110) um die Längsachse des Endoskops (10; 100) verdrehbar ist, wobei ferner in dem Endoskopgehäuse (18; 102) eine optisch abbildende Anordnung (42; 118) aufgenommen ist, die zur Fokussierung der Bildübertragung über eine Stelleinrichtung ( 60; 140) zumindest teilweise lageverstellbar ist, **dadurch gekennzeichnet, daß** das Endoskopgehäuse (18; 102) durchgehend hermetisch geschlossen ausgebildet ist, und daß das Drehgelenk (30; 115) außerhalb des Endoskopgehäuses angeordnet und am Fassungsanschluß (14; 108) ausgebildet ist.

2. Endoskop nach Anspruch 1, **dadurch gekennzeichnet, daß** das Drehgelenk (30; 115) im Bereich des proximalen Endes des Endoskopgehäuses (18; 102) angeordnet ist.

3. Endoskop nach Anspruch 1 oder 2, **dadurch gekennzeichnet, daß** der Fassungsanschluß (14; 108) an seinem distalen Ende eine auf dem Endoskopgehäuse umfänglich angeordnete und drehbar gelagerte Hülse (32) aufweist.

4. Endoskop nach einem der Ansprüche 1 bis 3, **dadurch gekennzeichnet, daß** das Endoskopgehäuse (18; 102) drehfest mit dem Endoskopschaft (12; 110) verbunden ist.

5. Endoskop nach einem der Ansprüche 1 bis 4, **dadurch gekennzeichnet, daß** die Stelleinrichtung (60; 140) von außen durch das geschlossene Endoskopgehäuse (18; 102) auf die optisch abbildende Anordnung (42; 118) wirkt.

6. Endoskop nach einem der Ansprüche 1 bis 5 **dadurch gekennzeichnet, daß** die Stelleinrichtung (60; 140) über einen magnetischen Kraftschluß auf die optisch abbildende Anordnung (42; 118) wirkt.

7. Endoskop nach einem der Ansprüche 1 bis 6, **dadurch gekennzeichnet, daß** die Stelleinrichtung (60; 140) einen Stellring (62) aufweist, der um die Außenseite des Endoskopgehäuses (18; 102) angeordnet ist und zur Lageverstellung zumindest eines Teils der optisch abbildenden Anordnung (42; 118) relativ zum Endoskopschaft (12; 110) verdrehbar ist.

8. Endoskop nach einem der Ansprüche 1 bis 6, **dadurch gekennzeichnet, daß** die Stelleinrichtung (60; 140) einen Stellring (142) aufweist, der zur Lageverstellung zumindest eines Teils der optisch abbildenden Anordnung (42; 118) relativ zum Fassungsanschluß (14; 108) verdrehbar ist.

9. Endoskop nach einem der Ansprüche 1 bis 8, **dadurch gekennzeichnet, daß** die Stelleinrichtung (60; 140) zumindest ein um die Außenseite des Endoskopgehäuses (18; 102) drehbar angeordnetes äußeres Ringelement (68; 148), das zumindest einen Magnet (70; 150) trägt, und zumindest ein innerhalb des Endoskopgehäuses (18; 102) angeordnetes drehbares inneres Ringelement (72; 152) aufweist, das ebenfalls zumindest einen Magnet (74; 154) trägt, wobei eine Drehung des äußeren Ringelementes (68; 148) das innere Ringelement (72; 152) bewegt und die Bewegung des inneren Ringelementes (72; 152) der Lageverstellung zumindest eines Teils der optisch abbildenden Anordnung (42; 118) dient.

10. Endoskop nach Anspruch 9, **dadurch gekennzeichnet, daß** die Bewegung des inneren Ringelementes (72; 152) über eine mechanische Verbindung in eine axiale Bewegung zumindest eines Teils der optisch abbildenden Anordnung (42; 118) umgesetzt wird.

11. Endoskop nach einem der Ansprüche 1 bis 10, **dadurch gekennzeichnet, daß** die Stelleinrichtung (140) zwei um die Außenseite des Endoskopgehäuses (102) angeordnete äußere Ringelemente (148, 162), die jeweils zumindest einen Magnet (150, 164) tragen, und zwei innerhalb des Endoskopgehäuses (102) angeordnete innere Ringelemente (152, 166) aufweist, die ebenfalls jeweils zumindest einen Magnet (154, 168) tragen, wobei eine Drehbewegung der äußeren Ringelemente (148, 162) das jeweilige zugehörige innere Ringelement (152, 166) bewegt, und wobei eine Drehung des einen äußeren Ringelementes (148) relativ zu dem anderen äußeren Ringelement (162) eine Lageverstellung zumindest eines Teils der optisch abbildenden Anordnung bewirkt.

12. Endoskop nach Anspruch 11, **dadurch gekennzeichnet, daß** das eine äußere Ringelement (162) drehfest mit dem Fassungsanschluß (108) verbunden ist und das andere äußere Ringelement (148) relativ zu diesem verdrehbar ist.

13. Endoskop nach Anspruch 12, **dadurch gekennzeichnet, daß** das nicht drehfest mit dem Fassungsanschluß (108) verbundene äußere Ringelement (148) mit dem Fassungsanschluß (108) reibschlüssig verbunden ist.

14. Endoskop nach einem der Ansprüche 1 bis 13, **dadurch gekennzeichnet, daß** die optisch abbildende Anordnung (42; 118) eine axial verschiebbare Okularlinsenanordnung (44; 120) des Endoskops (10; 100) und eine Objektivlinsenanordnung (46; 122) des optischen Aufnahmegerätes (16; 124) umfaßt, und daß mittels der Stelleinrichtung (60; 140) zur Fokussierung die Okularlinsenanordnung (44; 120) verschoben wird.

15. Endoskop nach einem der Ansprüche 1 bis 14, **dadurch gekennzeichnet, daß** die optisch abbildende Anordnung (42; 118) drehfest in dem Endoskopgehäuse (18; 102) angeordnet ist.

16. Endoskop nach einem der Ansprüche 1 bis 15, **dadurch gekennzeichnet, daß** in dem Endoskopgehäuse (18; 102) eine relativ zu diesem drehfeste Blende (82; 134) angeordnet ist, die eine Markierung zur Bestimmung der Drehlage des Fassungsanschlusses (14; 108) relativ zum Endoskopschaft (12; 110) aufweist.

17. Endoskop nach Anspruch 16, **dadurch gekennzeichnet, daß** die Blende (82; 134) in einer Zwischenbildebene angeordnet ist und zusammen mit der Okularlinsenanordnung (44; 120) verschoben wird.

## Claims

1. An endoscope, in particular a video endoscope, comprising an endoscope shaft (12; 110) at the distal end and a mount (14; 108) at the proximal end for connecting an optical imaging device (16; 124), for example a video camera, and comprising an endoscope housing (18; 102) between the endoscope shaft (12; 110) and the mount (14; 108), the endoscope housing (18; 102) being directly joined with the mount (14; 108) and the mount (14; 108) being rotatable relative to the endoscope shaft (12; 110), via a rotary joint (30; 115), about the longitudinal axis of the endoscope (10; 100), an optically imaging arrangement (42; 118), which is at least partially positionally displaceable by way of an adjusting device (60; 140) for focusing the image transmission, moreover being received in the endoscope housing (18; 102), **characterized in that** the endoscope housing (18; 102) is configured in continuously hermetically sealed fashion, and the rotary joint (30; 115) is arranged outside the endoscope housing and formed at the mount (14; 108).

2. The endoscope of Claim 1, **characterized in that** the rotary joint (30; 115) is arranged in the region of the proximal end of the endoscope housing (18; 102).

3. The endoscope of Claim 1 or 2, **characterized in that** the mount (14; 108) has at its distal end a sleeve (32) that is arranged circumferentially and mounted rotatably on the endoscope shaft.

4. The endoscope of anyone of Claims 1 through 3, **characterized in that** the endoscope housing (18; 102) is joined nonrotatably to the endoscope shaft (12; 110).

5. The endoscope of anyone of Claims 1 through 4, **characterized in that** the adjusting device (60; 140) acts on the optically imaging arrangement (42; 118) from outside through the sealed endoscope housing (18; 102).

6. The endoscope of anyone of Claims 1 through 5, **characterized in that** the adjusting device (60; 140) acts on the optically imaging arrangement (42; 118) via a magnetic non-positive engagement.

7. The endoscope of anyone of Claims 1 through 6, **characterized in that** the adjusting device (60; 140) has an adjusting ring (62) that is arranged around the outer side of the endoscope housing (18; 102) and is rotatable relative to the endoscope shaft (12; 110) for positional displacement of at least a portion of the optically imaging arrangement (42; 118).

8. The endoscope of anyone of Claims 1 through 6, **characterized in that** the adjusting device (60; 140) has an adjusting ring (142) that is rotatable relative to the mount (14; 108) for positional displacement of at least a portion of the optically imaging arrangement (42; 118).

9. The endoscope of anyone of Claims 1 through 8, **characterized in that** the adjusting device (60; 140) has at least one outer ring element (68; 148), arranged rotatably around the outer side of the endoscope housing (18; 102), that carries at least one magnet (70; 150), and at least one rotatable inner ring element (72; 152), arranged inside the endoscope housing (18; 102), that also carries at least one magnet (74; 154), wherein a rotation of the outer ring element (68; 148) moves the inner ring element (72; 152), and the movement of the inner ring element (72; 152) serves to positionally displace at least a portion of the optically imaging arrangement (42; 118).

10. The endoscope of Claim 9, **characterized in that** the movement of the inner ring element (72; 152) is converted via a mechanical connection into an axial movement of at least a portion of the optically imaging arrangement (42; 118).

11. The endoscope of anyone of Claims 1 through 10, **characterized in that** the adjusting device (140) has two outer ring elements (148, 162), arranged around the outer side of the endoscope housing (102), that each carry at least one magnet (150, 164), and two inner ring elements (152, 166), arranged inside the endoscope housing (102), that also each carry at least one magnet (154, 168), wherein a rotary movement of the outer ring elements (148, 162) moves the respective associated inner ring element (152, 166), and wherein a rotation of the one outer ring element (148) relative to the other outer ring element (162) effects a positional displacement of at least a portion of the optically imaging arrangement.

12. The endoscope of Claim 11, **characterized in that** the one outer ring element (162) is joined nonrotatably to the mount (108), and the other outer ring element (148) is rotatable relative to the latter.

13. The endoscope of Claim 12, **characterized in that** the outer ring element (148) that is not joined nonrotatably to the mount (108) is joined in frictionally engaged fashion to the mount (108).

14. The endoscope of anyone of Claims 1 through 13, **characterized in that** the optically imaging arrangement (42; 118) comprises an axially shiftable eyepiece lens arrangement (44; 120) of the endoscope (10; 100) and an objective lens arrangement (46; 122) of the optical imaging device (16; 124), and for focusing, the eyepiece lens arrangement (44; 120) is shifted by way of the adjusting device (60; 140).

15. The endoscope of anyone of Claims 1 through 14, **characterized in that** the optically imaging arrangement (42; 118) is arranged nonrotatably in the endoscope housing (18; 102).

16. The endoscope of anyone of Claims 1 through 15, **characterized in that** an aperture (82; 134) that has a marking for determining the rotational position of the mount (14; 108) relative to the endoscope shaft (12; 110) is arranged in the endoscope housing (18; 102) nonrotatably relative thereto.

17. The endoscope of Claim 16, **characterized in that** the aperture (82; 134) is arranged in an intermediate image plane and is shifted together with the eyepiece lens arrangement (44; 120).

## Revendications

1. Endoscope, en particulier endoscope vidéo, comportant une tige d'endoscope (12 ; 110) à l'extrémité distale et un raccord de monture (14 ; 108) à l'extrémité proximale pour raccorder un appareil optique de prise de vue (16 ; 124), par exemple une caméra vidéo, et comportant un boîtier d'endoscope (18 ; 102) entre la tige d'endoscope (12 ; 110) et le raccord de monture (14; 108), dans lequel le boîtier d'endoscope (18; 102) est directement relié au raccord de monture (14 ; 108) et le raccord de monture (14 ; 108) peut tourner, au moyen d'une articulation tournante (30 ; 115) par rapport à la tige d'endoscope (12 ; 110), autour de l'axe longitudinal de l'endoscope (10 ; 100), dans lequel en outre il est logé dans le boîtier d'endoscope (18 ; 102) un dispositif (42 ; 118) de reproduction optique dont la position est au moins partiellement réglable, au moyen d'un dispositif de réglage (60 ; 140) pour la mise au point de la transmission d'image, **caractérisé en ce que** le boîtier d'endoscope (18 ; 102) est réalisé hermétiquement fermé d'un bout à l'autre, et **en ce que** l'articulation tournante (30 ; 115) est disposée à l'extérieur du boîtier d'endoscope et est réalisée sur le raccord de monture (14 ; 108).

2. Endoscope selon la revendication 1, **caractérisé en ce que** l'articulation tournante (30 ; 115) est disposée dans la zone de l'extrémité proximale du boîtier d'endoscope (18 ; 102).

3. Endoscope selon la revendication 1 ou 2, **caractérisé en ce que** le raccord de monture (14 ; 108) comporte, à son extrémité distale, une douille (32) disposée périphériquement sur le boîtier d'endoscope et montée tournante.

4. Endoscope selon l'une des revendications 1 à 3, **caractérisé en ce que** le boîtier d'endoscope (18 ; 102) est lié solidairement en rotation à la tige d'endoscope (12 ; 110).

5. Endoscope selon l'une des revendications 1 à 4, **caractérisé en ce que** le dispositif de réglage (60 ; 140) agit de l'extérieur, à travers le boîtier d'endoscope (18 ; 102) fermé, sur le dispositif optique de reproduction (42 ; 118).

6. Endoscope selon l'une des revendications 1 à 5, **caractérisé en ce que** le dispositif de réglage (60 ; 140) agit, par un flux de forces magnétiques, sur le dispositif optique de reproduction (42 ; 118).

7. Endoscope selon l'une des revendications 1 à 6, **caractérisé en ce que** le dispositif de réglage (60 ; 140) comporte une bague de réglage (62) qui est disposée autour du côté extérieur du boîtier d'endoscope (18 ; 102) et peut être tournée par rapport à la tige d'endoscope (12 ; 110), pour le réglage de la position d'au moins une partie du dispositif optique de reproduction (42 ; 118).

8. Endoscope selon l'une des revendications 1 à 6, **caractérisé en ce que** le dispositif de réglage (60 ; 140) comporte une bague de réglage (142) qui peut être tournée par rapport au raccord de monture (14 ; 108) pour le réglage de la position d'au moins une partie du dispositif optique de reproduction (42 ; 118).

9. Endoscope selon l'une des revendications 1 à 8, **caractérisé en ce que** le dispositif de réglage (60 ; 140) comporte au moins un élément annulaire extérieur (68 ; 148) qui peut tourner autour du côté extérieur du boîtier d'endoscope (18 ; 102) et qui porte au moins un aimant (70 ; 150), ainsi qu'au moins un élément annulaire intérieur (72 ; 152) monté tournant à l'intérieur du boîtier d'endoscope (10 ; 102) et qui porte également au moins un aimant (74 ; 154), une rotation de l'élément annulaire extérieur (68 ; 148) déplaçant l'élément annulaire intérieur (72 ; 52) et le déplacement de l'élément annulaire intérieur (72 ; 152) servant au réglage de la position d'au moins une partie du dispositif optique de reproduction (42 ; 118).

10. Endoscope selon la revendication 9, **caractérisé en ce que** le déplacement de l'élément annulaire intérieur (72 ; 152) est converti, par une liaison mécanique, en un déplacement axial d'au moins une partie du dispositif optique de reproduction (42 ; 118).

11. Endoscope selon l'une des revendications 1 à 10, **caractérisé en ce que** le dispositif de réglage (140) comporte deux éléments annulaires extérieurs (148, 162), disposés autour du côté extérieur du boîtier d'endoscope (102) et qui portent chacun au moins un aimant (150, 164), ainsi que deux éléments annulaires intérieurs (152, 166) disposés à l'intérieur du boîtier d'endoscope (102), qui portent également chacun au moins un aimant (154, 168), un mouvement de rotation des éléments annulaires extérieurs (148, 162) déplaçant l'élément annulaire intérieur (152, 166) correspondant, et une rotation d'un élément annulaire extérieur (148) par rapport à l'autre élément annulaire extérieur (162) provoquant un réglage de la position d'au moins une partie du dispositif optique de reproduction.

12. Endoscope selon la revendication 11, **caractérisé en ce qu'**un élément annulaire extérieur (162) est lié solidairement en rotation au raccord de monture (108) et l'autre élément annulaire extérieur (148) peut être tournée par rapport à celui-ci.

13. Endoscope selon la revendication 12, **caractérisé en ce que** l'élément annulaire extérieur (148), non lié solidairement en rotation au raccord de monture (108), est lié par friction au raccord de monture (108).

14. Endoscope selon l'une des revendications 1 à 13, **caractérisé en ce que** le dispositif optique de reproduction (42; 118) comprend un agencement de lentilles d'oculaire (44 ; 120), pouvant coulisser axialement, de l'endoscope (10 ; 100) ainsi qu'un agencement de lentilles d'objectif (46 ; 122) de l'appareil optique de prise de vue (16 ; 124), et **en ce qu'**au moyen du dispositif de réglage (60 ; 140) l'agencement de lentilles d'oculaire (44 ; 120) est déplacé pour la mise au point.

15. Endoscope selon l'une des revendications 1 à 14, **caractérisé en ce que** le dispositif optique de reproduction (42 ; 118) est disposé de manière à ne pas pouvoir tourner dans le boîtier d'endoscope (18 ; 102).

16. Endoscope selon l'une des revendications 1 à 15, **caractérisé en ce que** dans le boîtier d'endoscope (18 ; 102) est disposé un diaphragme (82 ; 134), solidaire en rotation avec celui-ci, qui comporte un marquage pour déterminer la position angulaire du raccord de monture (14 ; 108) par rapport à la tige d'endoscope (12 ; 110).

17. Endoscope selon la revendication 16, **caractérisé en ce que** le diaphragme (82 ; 134) est disposé dans un plan d'image intermédiaire et est déplacé avec l'agencement de lentilles d'oculaire (44 ; 120).
